Europäisches Patentamt

European Patent Office

Office européen des brevets

(11) Publication number: **0 456 844 A1**

## EUROPEAN PATENT APPLICATION

(21) Application number: **90107773.5**

(22) Date of filing: **24.04.90**

(51) Int. Cl.⁵: **A61B 5/0225**

(43) Date of publication of application:
**21.11.91 Bulletin 91/47**

(84) Designated Contracting States:
**DE FR GB NL**

(71) Applicant: **Nihon Kohden Corporation**
**31-4, 1-chome, Nishiochiai**
**Shinjuku-ku Tokyo(JP)**

(72) Inventor: **Ogawa, Keikitsu**
**No. 7-23, Nakaarai 5-chome**
**Tokorozawa-shi, Saitama-ken(JP)**
Inventor: **Takeda, Suano**
**No. 23-10 Honshio**

**Ichikawa-shi, Chiba-ken(JP)**
Inventor: **Hyogo, Mitsushi**
**No. 10-7 Hatanaka 1-chome**
**Niiza-shi, Saitama-ken(JP)**
Inventor: **Shindo, Yoshiaki**
**No. 6-56, Maesawa 3-chome**
**Higashikurume-shi, Tokyo(JP)**

(74) Representative: **Sajda, Wolf E., Dipl.-Phys. et al**
**MEISSNER, BOLTE & PARTNER**
**Widenmayerstrasse 48 Postfach 86 06 24**
**W-8000 München 86(DE)**

(54) **Non-invasive automatic blood pressure measuring apparatus.**

(57) A non-invasive automatic blood pressure measuring apparatus comprises a blood flow meter (2) for non-invasively detecting the blood flow in arteries at the proximal or distal side of a cuff (1), a blood flow recovery point detecting means (3) detecting the recovery point of the blood flow signal output from the blood flow meter (2) during the depressing process of the internal pressure in the cuff (1), which has been compressed previously, and a blood flow returning point detecting means (4) detecting the returning point of the blood flow signal to the steady state in the further successive depressing process, and detecting the internal pressure in the cuff (1) at the recovery point (TH) as the maximum value of the blood pressure and the internal pressure in the cuff (1) at the returning point (TL) as the minimum value of the blood pressure.

FIG. 1

## Background of the Invention

### Field of the Invention

The present invention relates to a non-invasive automatic blood pressure measuring apparatus for non-invasively detecting the maximal (systolic) blood pressure and the minimal (diastolic) blood pressure in a blood pressure measuring process, comprising attaching a cuff at a part of the body, compressing the attached cuff by pumping or the like, and depressing again the internal pressure of the cuff, while measuring characteristic values.

### Description of the Prior Art

For such an automatic blood pressure measuring apparatus, there are two kinds of apparatus, namely, a first type which detects Korotkov sounds by a microphone in the depressing process and which measures the internal pressure of the cuff at the start and at the termination of these sounds according to the Korotkov sound recognizing method; and a second type which detects a pulse wave caused by the pulsation of an artery as a vibration upon the internal pressure of the cuff and which measures the blood pressure based on the change of this vibration according to an oscillometric method.

However, the apparatus of the first method is easily affected by noises. In addition, there is a problem in its measuring precision because there are cases in which Korotkov sounds sometimes fade away or do not disappear even if blood pressure is minimal.

In the second method, although the problems caused by the lack of stability of the generation of Korotkov sounds have mainly been solved, there still remains a problem with its measuring precision. Since the pulse wave is detected as an internal pressure change in the cuff, the pulse waves contacting the cuff at different points have a variation in the direction of the width of the cuff. So, these varied values are detected and are added and superposed during operation. In addition, since the detection of minimal blood pressure from the vibration itself is difficult, and the respective value is supposed from the operation using the mean value of the blood pressure, a problem also remains in its measuring precision.

### Summary of the Invention

The object of the present invention is to provide a non-invasive automatic blood pressure measuring apparatus based on a new system which is capable of measuring blood pressure with greater accuracy.

To accomplish this purpose, the non-invasive automatic blood pressure measuring apparatus of the present invention detects a recovery point of a blood flow signal output from a blood flow meter in the depressing process of the internal pressure of the cuff, which was previously compressed to a specified pressure, holds the internal pressure of the cuff at this recovery point at the time at which the blood flow is at the maximum value of the blood pressure, detects a returning point at the time at which the blood flow signal reaches the steady state in the depressing process, and holds the internal pressure of the cuff of this returning point at the time at which the blood flow is at the minimum value of the blood pressure.

According to the present invention, both maximal and minimal blood pressures can be measured with high accuracy since the values of both blood pressures can be determined directly from the pulse signals whose vibrating amplitude is more correctly correlated with the depression of the internal pressure of the cuff and which has a high S/N ratio and stability, by non-invasively detecting and signal processing of the blood flow.

### Brief Description of the Drawings

The invention will be described in detail by means of preferred embodiments and with reference to the accompanying drawings.

Fig. 1 shows the structure of the non-invasive automatic blood measuring apparatus of an embodiment in order to explain the present invention in principle.

Fig. 2 shows graphs for explaining the operation of the embodiment according to Fig. 1.

Fig. 3 shows a detailed structure of the non-invasive automatic blood pressure measuring apparatus of an embodiment of the present invention.

Fig. 4 shows graphs for explaining the operation of the embodiment according to Fig. 3.

### Description of the Preferred Embodiments

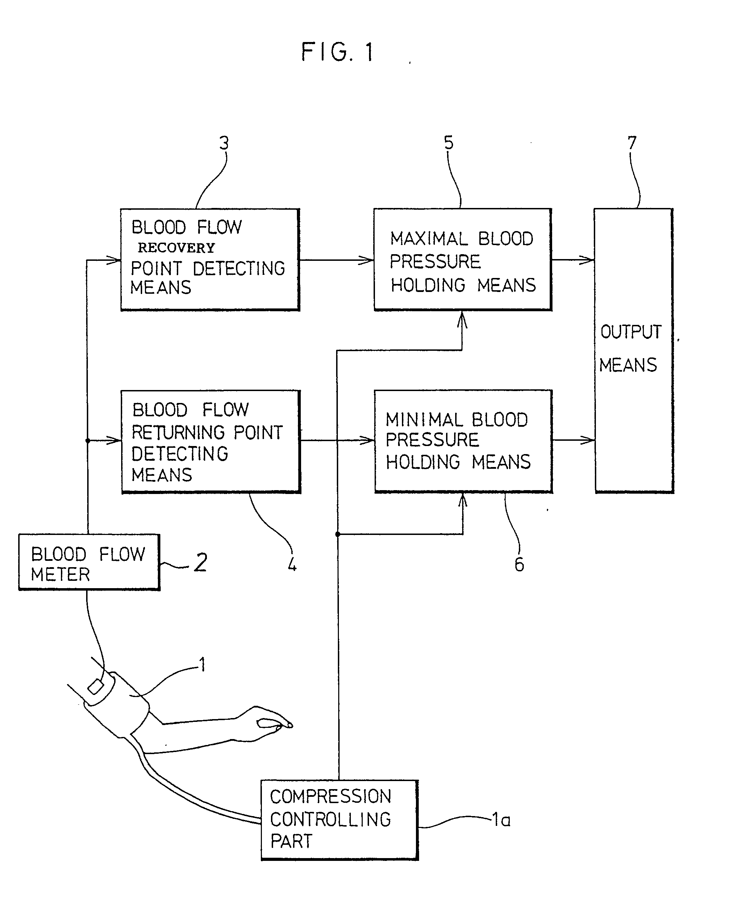

Fig. 1 shows a diagrammatic view of an embodiment in order to explain the principle of the present invention.

As shown in this Fig. 1, a non-invasive automatic blood pressure measuring apparatus of this embodiment comprises the following components: a cuff 1 which is attached to a part of a body, compressed by a compression controlling part 1a and thereafter depressed for measuring purposes; a blood flow meter 2 non-invasively detecting the flow of blood in the arteries of the part of the body close to the cuff 1 at the proximal or distal side of this cuff 1;

a blood flow recovery point detecting means 3 detecting a recovery point of the blood signal output from the blood flow meter 2 in the depressing process of the internal pressure in the cuff 1, caused after a standstill of the flow due to complete compression of the arteries when the pressure has been released;

a blood flow returning point detecting means 4 detecting the returning point of the blood flow signal to the steady state during the further successive depressing process wherein the compressive force of the cuff 1 has ceased;

a maximal blood pressure holding means 5 holding the internal pressure in the cuff 1 at the recovery point as the maximum value of the blood pressure;

a minimal blood pressure holding means 6 holding the internal pressure in the cuff 1 at the returning point as the minimum value of the blood pressure; and

an output means 7 displaying and/or printing out these maximal and minimal holding values of the blood pressure.

As for the blood flow recovery point and the blood flow returning point detecting means 3 and 4, respectively, it is proposed to detect the recovery point and the returning point of the blood pressure from the blood flow signal wherein the pulsation component of the blood signal has been smoothed and standardized out of its maximum and minimum values.

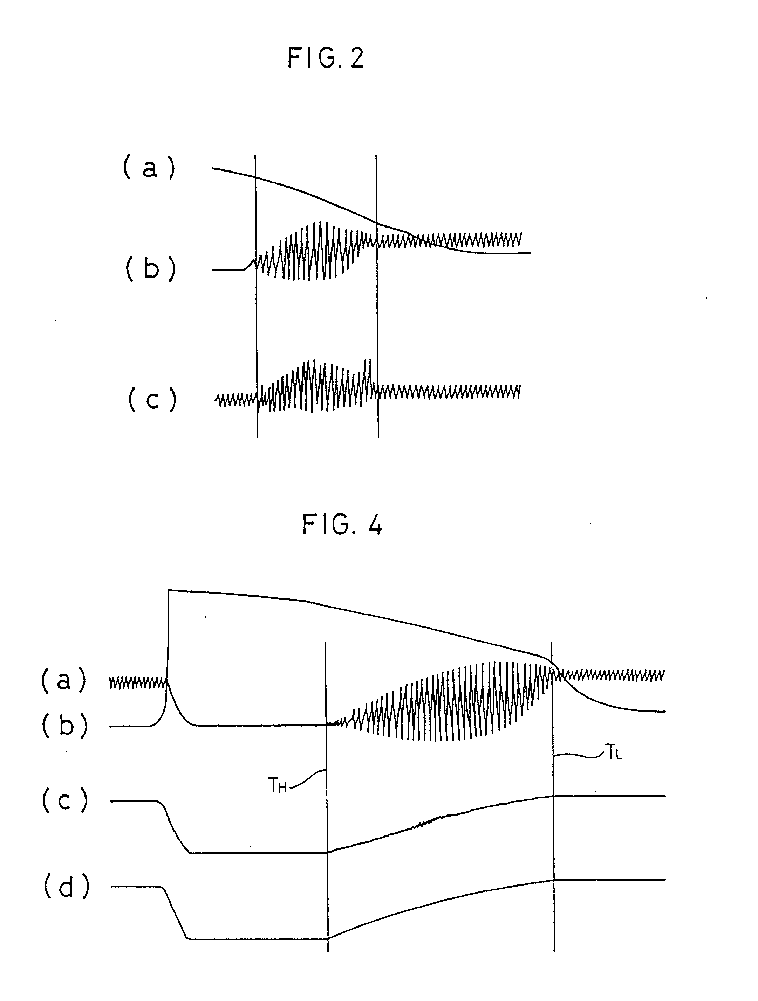

When measuring the blood pressure, the cuff 1 is gradually depressed for measuring the blood pressure after it has been compressed by the compression controlling part 1a. As to this depressing process, the decreasing internal pressure of the cuff 1 is schematically shown in Fig. 2(a).

At this time, the blood flow signal according to Fig. 2(b) and detected by the blood flow meter 2 increased gradually in its vibration amplitude from the interruption or standstill of the blood flow, and its vibration amplitude reaches its maximum in the average blood pressure region. Gradually, the vibration amplitude reaches its steady state in which the pulsations of a constant vibration amplitude are repeated in a normal flow direction when the depression is further continued.

The blood flow recovery point detecting means 3 detects the recovery point of the blood flow signal indicating the initiation of the blood flow from the standstill and causes the maximal blood pressure holding means 5 to hold the pressure of the internal pressure in the cuff 1 as the maximum value of the blood pressure.

On the other hand, the blood flow returning point detecting means 4 detects the returning point where the vibrating amplitude returns to its steady state as the blood flow returning point, causes the minimal blood pressure holding means 6 to hold the minimum value of the blood pressure and causes the output means 7 to output and/or display them.

Incidentally, the pulsation caused by the vibration of the internal pressure in the cuff 1 according to the oscillometric method is inferior in the S/N ratio and in the correlativity between the change in the internal pressure in the cuff 1 when compared with the blood flow signal, as shown in Fig. 2(c).

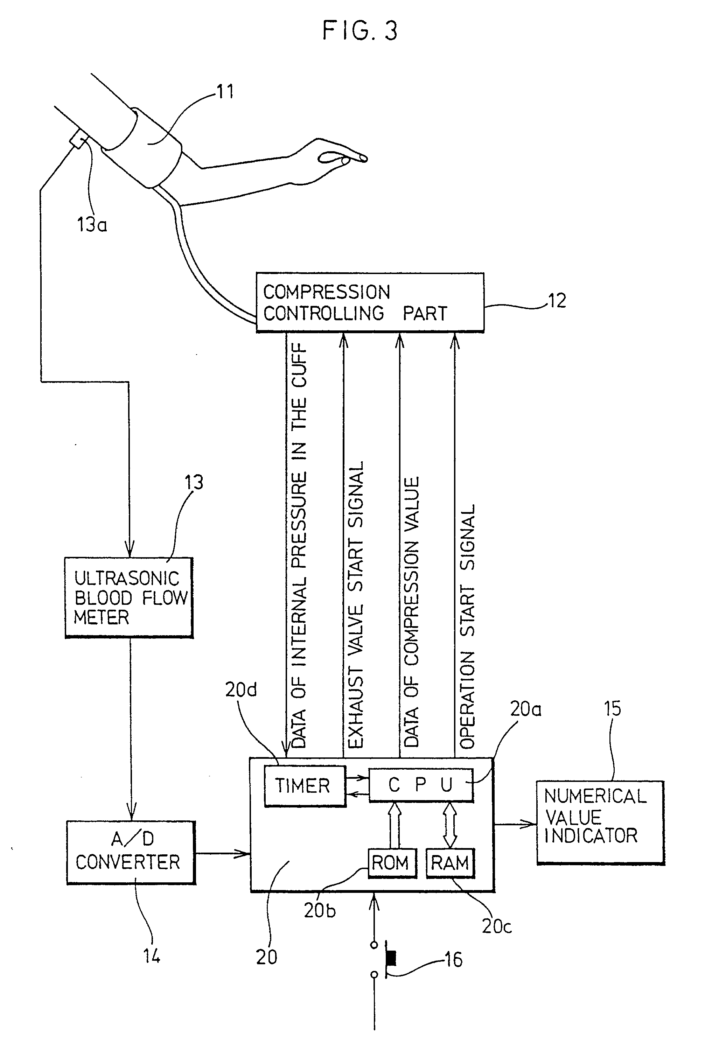

Fig. 3 shows a circuit diagram of a non-invasive automatic blood pressure measuring apparatus according to a further embodiment of the present invention wherein a microcomputer 20 is used. In this Fig. 3, a cuff 11 attached for example to the upper arm of a person is compressed and depressed, respectively, by a compression controlling part 12 as is generally known.

Reference numeral 13 denotes an ultrasonic blood flow meter having a probe or sensor 13a set at the upper arm to which the proximal side of the cuff 11 is adjacent. Reference numeral 14 denotes an A/D converter digitizing the blood flow signal, and a numerical value indicator 15 is used for indicating the maximal and minimal blood pressures, respectively. A recorder can be used in addition to or instead of this numerical value indicator 15 as output means. Reference numeral 16 denotes a starting switch.

After compressing the air to a specified pressure with a pressure pump according to the instructions from the microcomputer 20, the compression controlling part 12 executes gradually exhausting and depresses the compressed air by controlling an exhaust valve. Then, it opens the exhaust valve completely according to further instructions which are sent from the microcomputer 20 when the minimal blood pressure has been detected. During this time, the data of the internal pressure in the cuff 11, detected by the built-in pressure sensor or probe 13a, are sent to the microcomputer 20.

The microcomputer 20 is operated by a CPU 20a according to a program stored in a ROM 20b, transmits and receives control signals or data to and/or from the aforementioned components 12, 14, 15 and 16, respectively, through a built-in input/output-port. So, the microcomputer 20 works as the blood flow recovery point and returning point detecting means as well as the maximal and minimal blood pressure holding means according to the present invention.

Eventually, a RAM 20c time-serially memorizes the amplitude data of the internal pressure in the cuff 11, see Fig. 4(a) and the detected data of the vibration detected by the sensor 13a and related with the internal pressure in the cuff 11, see Fig. 4-(b). Then the CPU 20a performs the following operating processing in accordance with the program stored in the ROM 20b taking in the timer signal of a timer 20d based on these memorized data.

That is, the CPU 20a first prepares the averaged amplitude data as shown in Fig. 4(c) by detecting the maximum and the minimum values of the amplitude data taken one after another, and causes the RAM 20c to memorize these averaged amplitude data by making them similarly correspond to the internal pressure in the cuff 11 with passing time.

Next, the CPU 20a prepares moving averaged amplitude data according to Fig. 4(d), for example, for 4 seconds at 0,1 second intervals. Then it causes the RAM 20c again to memorize these moving averaged data by making them correspond to the internal pressure in the cuff 11 with passing time. Furthermore, the CPU 20a also prepares the moving averaged amplitude data before initiating the compression, calculates its 1% value and sends the operation start signal to the compression controlling part 12.

Then, the CPU 20a detects the time at which the moving averaged data in the depresseion process rise from the standstill to the aforementioned 1% value as the blood flow recovering point TH, holds the internal pressure in the cuff 11 during this time, and causes the indicator 15 to indicate this pressure at TH as the maximal blood pressure.

Next, the CPU 20a prepares and delivers the increase rate of the moving averaged data of the blood flow signal in the successive depression process, i.e., the differential data, and measures the time that it takes for this increase rate to decrease to 1% of its maximum value, as the blood flow returning time TL, while the amplitude of the detected data increases, holds the internal pressure in the cuff 11 during this time, and causes the indicator 15 to indicate this pressure at TL as the minimal blood pressure.

Next, the operation of the non-invasive automatic blood pressure measuring apparatus having such a construction will be explained in detail.

An ultrasonic blood flow meter 13 non-invasively detects the blood flow when its probe or sensor 13a is applied to an artery part on the distal side of the cuff 11. When the start switch 16 is turned on, the microcomputer 20 is initialized, takes in the digitized blood flow signal before the initiation of compression and prepares the moving averaged amplitude data.

Also, the microcomputer 20 initiates the depression when the cuff 1 has been compressed to the specified compression value taking the specified time. The microcomputer 20 performs averaging the maximum and minimum values of the vibrating blood flow signal and moving averaging these averaged data in this depressing process as described above in connection with Fig. 4(a) to 4-(d).

In parallel with this operation process, the microcomputer 20 further detects the blood flow recovery point TH and the blood flow returning point TL with the aforementioned processing method, detects and holds their corresponding internal pressures in the cuff 11 as the maximal (systolic) and the minimal (diastolic) blood pressures, respectively, and causes the indicator 15 to indicate them. After detection of the minimal blood pressure, it rapidly decreases the internal pressure in the cuff 11 by opening the exhaust valve completely, and terminates the measuring process.

According to the blood pressure measurement of this blood flow detecting system, the maximal (systolic) and the minimal (diastolic) blood pressures can be detected with high precision directly from the blood flow signal because the amplitude of the pulsation signal changes smoothly corresponding to the internal pressure in the cuff 11, and it imitates the depression of the internal pressure in the cuff 11 with high precision by the moving average.

As for the blood flow detecting system of the present invention, it is proposed that the system can also directly prepare the envelope curve with moving averaging of the maximum and minimum values of the vibrating waveform of the blood flow signal, for example by 5 successive pulsation data, without smoothing them once, detect the time that they exceed the slightly increasing envelope curve level determined in advance from the standstill as the recovery point of the blood flow and detect the time when they return to the slightly increasing envelope curve level determined in advance from the envelope curve level from the large pulsation of the average blood pressure region to the steady state as the returning point of the blood flow.

## Claims

1. A non-invasive automatic blood pressure measuring apparatus, comprising:
   - a cuff (1, 11) attachable to a part of a body and being compressible, which after compression to a specified value of the internal pressure is depressed for measuring purposes;
   - a blood flow meter (2, 13) non-invasively detecting the blood flow in arteries at the proximal or distal side of the cuff (1, 11);
   - a blood flow recovery point detecting means (3) for detecting the recovery point of a blood flow signal output from the blood flow meter (2, 13) in the depressing process of the internal pressure of the cuff (1, 11);

- a blood flow returning point detecting means (4) for detecting the returning point of the blood flow signal to the steady state in the further successive depressing process;

- a maximal blood pressure holding means (5) for holding the internal pressure in the cuff (1, 11) of the recovery point (TH) as the maximal blood pressure;

- a minimal blood pressure holding means (6) for holding the internal pressure in the cuff (1, 11) of the returning point (TL) as the minimal blood pressure; and

- an output means (7, 15) for indicating or printing out these held maximal and minimal blood pressures.

2. The non-invasive automatic blood pressure measuring apparatus according to claim 1, wherein the blood flow recovery point detecting means (3) and the blood flow returning point detecting means (4) detect a blood flow recovery point and a blood flow returning point from an averaged blood flow signal obtained after smoothing the pulsation component of the blood flow signal.

3. The non-invasive automatic blood pressure measuring apparatus according to claim 1, wherein the blood flow recovery point detecting means (3) and the blood flow returning point detecting means (4) detect a blood flow recovery point and a blood flow returning point from a maximum and a minimum value of the pulsation component of the blood flow signal.

FIG. 1

# FIG. 2

( a )

( b )

( c )

# FIG. 4

( a )

( b )

$T_H$

$T_L$

( c )

( d )

# FIG. 3

COMPRESSION CONTROLLING PART — 12

ULTRASONIC BLOOD FLOW METER — 13

A/D CONVERTER — 14

DATA OF INTERNAL PRESSURE IN THE CUFF

EXHAUST VALVE START SIGNAL

DATA OF COMPRESSION VALUE

OPERATION START SIGNAL

TIMER

C P U

ROM

RAM

NUMERICAL VALUE INDICATOR — 15

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 105 021   (W.J. WILLIAMS et al.)<br>* column 2, line 40 column 4, line 36; figures 1,2 * | 1 | A 61 B 5/0225 |
| A | | 2,3 | |
| | — — — | | |
| A | US-A-4 712 564   (KEIJI YAMAGUCHI)<br>* column 3, line 50 - column 4, line 17; column 5, lines 23-38; figure 1 * | 1 | |
| | — — — | | |
| A | US-A-4 660 566   (YORAM PALTI)<br>* column 11, line 1 - column 12, line 14; figures 12-16 * | 1 | |
| | — — — — — | | |

TECHNICAL FIELDS
SEARCHED (Int. Cl.5)

A 61 B 5/00
A 61 B 8/00

The present search report has been drawn up for all claims

| Place of search | Date of completion of search | Examiner |
|---|---|---|
| Berlin | 14 December 90 | WEIHS J.A. |

CATEGORY OF CITED DOCUMENTS

X : particularly relevant if taken alone
Y : particularly relevant if combined with another
document of the same catagory
A : technological background
O : non-written disclosure
P : intermediate document
T : theory or principle underlying the invention

E : earlier patent document, but published on, or after
the filing date
D : document cited in the application
L : document cited for other reasons

&: member of the same patent family, corresponding
document